(19) Europäisches Patentamt
European Patent Office
Office européen des brevets

(11) EP 1 184 367 A1

(12) EUROPEAN PATENT APPLICATION
published in accordance with Art. 158(3) EPC

(43) Date of publication:
**06.03.2002 Bulletin 2002/10**

(21) Application number: **00917319.6**

(22) Date of filing: **14.04.2000**

(51) Int Cl.[7]: **C07C 237/32**, C07D 213/81, A61K 31/216, A61K 31/222, A61K 31/44, A61K 31/166, A61P 25/28, A61P 25/00, A61P 25/16, A61P 25/14, A61P 25/08, A61P 27/06, A61P 27/08, C07D 237/24, C07D 239/28

(86) International application number:
**PCT/JP00/02470**

(87) International publication number:
**WO 00/64859 (02.11.2000 Gazette 2000/44)**

(84) Designated Contracting States:
**AT BE CH CY DE DK ES FI FR GB GR IE IT LI LU MC NL PT SE**
Designated Extension States:
**AL LT LV MK RO SI**

(30) Priority: **21.04.1999 JP 11373899**

(71) Applicant: **Sumitomo Pharmaceuticals Company, Limited**
**Osaka-shi, Osaka 541-8510 (JP)**

(72) Inventors:
- **NISHIHARA, Toshio**
  **Nishinomiya-shi, Hyogo 662-0831 (JP)**
- **MURAOKA, Masami**
  **Toyonaka-shi, Osaka 560-0053 (JP)**

(74) Representative: **Cresswell, Thomas Anthony et al**
**J.A. Kemp & Co. 14 South Square Gray's Inn**
**London WC1R 5LX (GB)**

(54) **AMIDE DERIVATIVES**

(57) A prodrug of the compound given by formula:

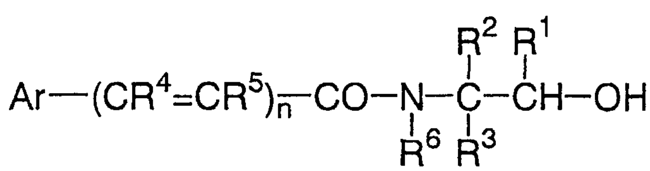

wherein Ar is an optionally substituted phenyl group or optionally substituted aromatic heterocyclic group; n is an integer of 0, 1 or 2;

$R^1$ is a hydrogen atom or an optionally substituted alkyl group; $R^2$ and $R^3$ are independently an optionally substituted alkyl group, or $R^2$ may be bonded with $R^1$ or $R^3$ and taken together with the carbon atom adjacent thereto to form an optionally substituted cycloalkane ring;
$R^4$ and $R^5$ are independently hydrogen atom or an optionally substituted alkyl group;
$R^6$ is a hydrogen atom, hydroxy group or alkyl group;

which is useful as a medicament for treating neurodegenerative disorders such as retinal neurodegenerative disorders and the like.

EP 1 184 367 A1

## Description

### Technical Field

The present invention relates to amide derivatives useful as a medicament and medicaments containing the amide derivative as an active ingredient for treating neurodegenerative disorders, especially retinal neurodegenerative disorders.

### Background Art

N-t-Butyl-benzamide, N-t-butyl-4-bromobenzamide, N-t-butyl-4-nitrobenzamide, etc. are known to be useful as a medicament for treating neurodegenerative disorders such as Parkinson's disease, multiple sclerosis, Alzheimer's disease and the like (WO 95/28153, WO 96/31462).

N-t-Butyl-3-chloro-2-pyridinecarboxamide, N-(2-hydroxy-1,1-dimethylethyl)-6-chloro-2-pyridinecarboxamide, etc. are known to be useful as a herbicide (JP 48-26918(A), JP 60-72803(A), JP 61-151174(A)).

It has been known that N-t-butyl-4-fluorobenzamide, N-t-butyl-2-fluorobenzamide, N-(2-hydroxy-1,1-dimethylethyl)-2,4,5-trifluorobenzamide, N-(2-hydroxy-1,1-dimethylethyl)-2,5-difluorobenzamide, N-(2-hydroxy-1,1-dimethylethyl)-2-fluorobenzamide, N-(2-hydroxy-1,1-dimethylethyl)-5-chloro-2-fluorobenzamide, N-(2-hydroxy-1,1-dimethylethyl)-2-fluoro-6-iodobenzamide, N-(2-hydroxy-1,1-dimethylethyl)-2,6-difluorobenzamide, N-(2-hydroxy-1,1-dimethylethyl)-2-chloro-4-fluorobenzamide, etc. were produced as synthetic intermediates and so on (EP 511073, WO 89/06649, J. Org. Chem., 52, 713(1987), J. Org. Chem., 53, 345(1988), EP 538231, US 3985889, etc.).

However, these documents do not disclose that the amide derivatives are effective for treating retinal neurodegenerative disorders.

### Disclosure of the invention

The present invention is intended to provide a medicament for treating neurodegenerative disorders, especially retinal neurodegenerative disorders.

The inventors of the present invention have intensively carried out research, and found that some amide derivatives are useful as a medicament for treating retinal neurodegenerative disorders and the like. Thus, the present invention has been accomplished.

Namely, the present inventions are disclosed in the following [1] - [4].

[1] A prodrug of an amide given by formula 1:

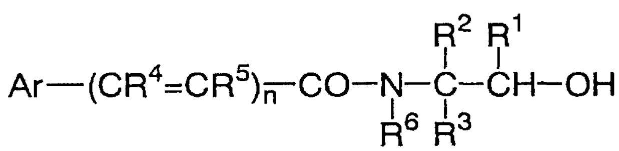

*(1)*

wherein Ar is an optionally substituted phenyl or optionally substituted aromatic heterocyclic group; n is an integer of 0, 1 or 2;

$R^1$ is a hydogen atom or an optionally substituted alkyl group; $R^2$ and $R^3$ are independently an optionally substituted alkyl group; or $R^2$ may be bonded with
$R^1$ or $R^3$ and taken together with the carbon atom adjacent thereto to form an optionally substituted cycloalkane ring;
$R^4$ and $R^5$ are independently a hydrogen atom or an optionally substituted alkyl group;
$R^6$ is a hydrogen atom, a hydroxy group or an alkyl group;
or a pharmaceutically acceptable salt thereof.

[2] A medicament for treating neurodegenerative disorders, comprising a compound described in [I] above.

[3] A medicament comprising a prodrug given by formula 2:

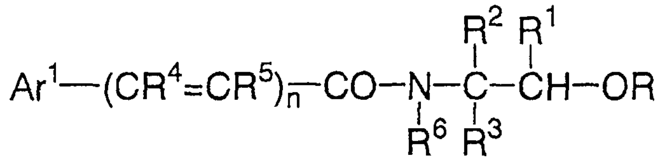

(2)

wherein $Ar^1$ is a phenyl group substituted by fluorine atom at 2- or 4-position (said phenyl group may be further substituted with one or two halogen atoms) or an optionally substituted 6-membered aromatic heterocyclic group; R is an acyl group, optionally substituted phosphono group or optionally substituted alkanoyloxymethyl group; and n, $R^1$, $R^2$, $R^3$, $R^4$, $R^5$ and $R^6$ are as defined above; or a pharmaceutically acceptable salt thereof.

[4] A prodrug given by formula 3:

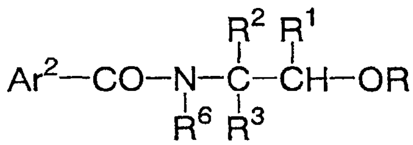

(3)

wherein $Ar^2$ is a group of formula:

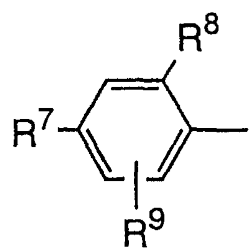

or a 6-membered aromatic heterocyclic group substituted with 1-3 halogen atoms; n, R, $R^1$, $R^2$, $R^3$ and $R^6$ are as defined above; one of $R^7$ and $R^8$ is a fluorine atom and the other is a hydrogen atom or a halogen atom; and $R^9$ is a hydrogen atom or a halogen atom;

or a pharmaceutically acceptable salt thereof.

In the present invention, "prodrug" means a compound modified its hydroxy group by a group that is removable *in vivo.* Typical examples of the modifying group include acyl group, optionally substituted phosphono group, optionally substituted alkanoyloxymethyl group and so on.

Examples of the "acyl group" include an optionally substituted-alkanoyl group, optionally substituted aroyl group, optionally substituted alkoxycarbonyl group, optionally substituted aminocarbonyl group and so on.

Examples of the "alkanoyl group" include a straight-chain or branched alkanoyl group having 20 or less carbon atoms, typically formyl, acetyl, propionyl, butyryl, isobutyryl, valeryl, isovaleryl, pivaloyl, 2-methylbutyryl, hexanoyl, lauroyl, myristoyl, palmitoyl, stearoyl and so on.

Examples of the "aroyl group" include a group having 10 or less carbon atoms such as benzoyl, toluoyl, naphthoyl and so on.

Examples of the "substituent" for the substituted alkanoyl group and substituted aroyl group include amino group, alkylamino group, dialkylamino group, alkanoylamino group, aroylamino group, carboxy group, alkoxycarbonyl group, aryloxycarbonyl group, hydroxyl group, alkoxy group, aryloxy group, halogen atom, cyano group, nitro group, alkylsulfonylamino group, arylsulfonylamino group, carbamoyl group and so on.

Examples of the "substituent" for the substituted alkoxycarbonyl group include alkyl group, arylalkyl group, aryl group and so on.

Examples of the "substituent" for the substituted aminocarbonyl group include alkyl group, arylalkyl group, aryl group and so on.

In the dialkylamino group, two alkyl groups, which are substituents on the amino group, may be bonded with each other or further intervened by oxygen atom, taken together with the nitrogen atom of the amino group to form a

5- or 6-membered saturated heterocyclic ring such as piperidyl, pyrrolidyl, morpholyl and so on.

Preferable acyl group includes alkanoyl group, substituted alkanoyl group (e.g. aminoalkanoyl group, carboxyalkanoyl group), aroyl group, optionally substituted alkoxycarbonyl group, optionally substituted aminocarbonyl group and so on.

Typical examples of the acyl group include formyl, acetyl, propionyl, butyryl, isobutyryl, valeryl, isovaleryl, pivaloyl, 2-methylbutyryl, hexanoyl, lauroyl, myristoyl, palmitoyl, stearoyl, 3-aminopropionyl, 3-dimethylaminopropionyl, 2-dimethylaminobutyryl, 3-dimethylaminobutyryl, 4-dimethylaminobutyryl, 5-dimethylaminovaleryl, ethylaminoacetyl, diethylaminoacetyl, propylaminoacetyl, (1-pyrrolidyl)acetyl, (1-piperidyl)acetyl, morpholinoacetyl, acyl residue of α -amino acids (e.g. glycyl, alanyl, phenylalanyl, arginyl, lysyl, α-aspartyl, β-aspartyl, α-glutamyl, γ-glutamyl, methylaminoacetyl, dimethylaminoacetyl, 2-dimethylaminopropionyl, 2-dimethylamino-2-methylpropionyl, etc.), carboxyacetyl, 2-carboxypropionyl, 3-carboxypropionyl, 2-carboxybutyryl, 3-carboxybutyryl, 4-carboxybutyryl, 2-carboxy-2-methylpropionyl, glycoloyl, lactoyl, benzoyl, 1-naphthoyl, 2-naphthoyl, 2-carboxybenzoyl, 3-carboxybenzoyl, 4-carboxybenzoyl, 2-hydroxybenzoyl, 2-aminobenzoyl, methoxycarbonyl, ethoxycarbonyl, propoxycarbonyl, t-butoxycarbonyl, benzyloxycarbonyl, aminocarbonyl, methylaminocarbonyl, dimethylaminocarbonyl, ethylaminocarbonyl, diethylaminocarbonyl, propylaminocarbonyl, butylaminocarbonyl, pyrrolidinocarbonyl, piperidinocarbonyl, morpholinocarbonyl and so on.

Examples of the "substituent" for the substituted phosphono group include alkyl group, arylalkyl group, aryl group and so on. Typical examples of optionally substituted phosphono group include phosphono, dimethylphosphono, diethylphosphono and so on.

When the "substituent" for the optionally substituted alkanoyloxymethyl group exists on the alkanoyl group, the substituent and the substituted alkanoyl group are exemplified by the same substituted alkanoyl group as described above. Further, substituents such as alkyl group may be on the "methyl" part of the alkanoyloxymethyl group.

Examples of the "aryl group" include a group having 10 or less carbon atoms such as phenyl, tolyl, naphthoyl and so on.

Examples of the "aromatic heterocyclic group" include 5- or 6-membered aromatic heterocyclic group containing 1-3 heteroatoms independently selected from the group of nitrogen, sulfur and oxygen atom. The nitrogen or sulfur atom constituting the ring may be oxidized. Examples of the 5-membered aromatic heterocyclic group include the 5-membered aromatic heterocyclic group containing 1 or 2 heteroatoms independently selected from the group of nitrogen, sulfur and oxygen atom, such as pyrrolyl, threnyl, furyl, imidazolyl, pyrazolyl, isothiazolyl, isoxazolyl, thiazolyl, oxazolyl and so on. Examples of the 6-membered aromatic heterocyclic group include the 6-membered aromatic heterocyclic group containing 1-3 nitrogen atoms, typically pyridyl, 1-oxide-pyridyl, pyrazinyl, pyrimidinyl, pyridazinyl, triazinyl and so on.

Examples of the "substituent" for the substituted phenyl group, substituted aromatic heterocyclic group, substituted 5-membered aromatic heterocyclic group and substituted 6-membered aromatic heterocyclic group include halogen atom, cyano group, nitro group, alkyl group, halogen-substituted alkyl group, alkoxy group, halogen-substituted alkoxy group, alkoxycarbonyl group, alkanoylamino group, amino group, phenyl group, alkylaminocarbonylamino group, alkoxycarbonylamino group, alkylsulfonylamino group, carbamoyl group, alkyl-substituted carbamoyl group and so on. The number of the substituent may be one, two or more independently from each of them.

Examples of preferable substituent for the substituted phenyl group include halogen atom, cyano group, nitro group, halogen-substituted alkyl group, halogen-substituted alkoxy group, alkoxycarbonyl group, alkanoylamino group, amino group, phenyl group, alkylaminocarbonylamino group, alkoxycarbonylamino group, alkylsulfonylamino group, carbamoyl group, alkyl-substituted carbamoyl group and so on. More preferable substituents are electron-withdrawing group such as halogen atom, cyano -group, nitro group, trifluoromethyl group and so on. Further, more preferable group is halogen atom, especially fluorine atom.

Examples of preferable substituent for the substituted aromatic heterocyclic group, substituted 5-membered aromatic heterocyclic group and substituted 6-membered aromatic heterocyclic group include halogen atom, cyano group, nitro group, alkyl group, halogen-substituted alkyl group, alkoxycarbonyl group, alkanoylamino group, amino group, phenyl group, alkylaminocarbonylamino group, alkoxycarbonylamino group, alkylsulfonylamino group, carbamoyl group, alkyl-substituted carbamoyl group and so on, especially halogen atom.

In the substituted phenyl, the number of the substituent is, for example, one, two or three, preferably one or two, more preferably two. Preferable position of the substituent is 4-position, or 2,4-position when substituted by plural substituents. In the substituted aromatic heterocyclic group, substituted 5-membered aromatic heterocyclic group and substituted 6-membered aromatic heterocyclic group, the number of the substituent is, for example, one, two or three, preferably one or two, more preferably one.

Examples of the "alkyl group" include a straight-chain or branched alkyl group having 6 or less carbon atoms, typically methyl, ethyl, propyl, 1-methylethyl, butyl, 2-methylpropyl, pentyl, 1,2-dimethylpropyl, hexyl, 3-methylpentyl and so on.

For an alkyl group which is a part of the other group, e.g., for the alkyl part of halogen-substituted alkyl group,

alkylaminocarbonyl group, alkylsulfonylamino group, alkylamino group, dialkylamino group and so on, there can be exemplified the above-mentioned alkyl groups. Further, it is also the same when a group explained in the present description is a part of the other group.

Examples of the "alkoxy group" include a straight-chain or branched alkoxy group having 6 or less carbon atoms, typically methoxy, ethoxy, propoxy, 1-methylethoxy, butoxy, 2-methylpropoxy, pentyloxy, 1,2-dimethylpropoxy, hexyloxy, 3-methylpentoxy and so on.

The "alkoxyalkoxy group" means an "alkoxy group" substituted with "alkoxy group." The "alkoxycarbonyl group" means a carbonyl group substituted with "alkoxy group."

The "halogen-substituted alkyl group" and "halogen substituted alkoxy group" mean "alkyl group" and "alkoxy group" substituted with one, two or more halogen atoms, respectively. Each of the preferable examples is, for example, trifluoromethyl and trifluoromethoxy, respectively.

The "alkyl-substituted carbamoyl group" contains monoalkylcarbamoyl group and dialkylcarbamoyl group, and examples of the alkyl part are above-mentioned alkyl group.

Examples of the "halogen atom" include fluorine, chlorine, bromine, iodine atom and so on, preferably fluorine, chlorine and bromine atom, more preferably fluorine atom.

Examples of the "cycloalkane ring" include a cycloalkane ring having 3-8 carbon atoms, typically cyclopropane, cyclobutane, cyclopentane, cyclohexane, cycloheptane, cyclooctane ring and so on.

Examples of the "substituent" for substituted alkyl and substituted cycloalkane ring include cycloalkyl, alkoxy, hydroxy group, halogen atom, alkoxyalkoxy, alkanoyloxy, amino, alkylamino, dialkylamino, alkanoylamino and so on, and further an aryl group such as phenyl, tolyl and so on, and heterocyclic group such as pyrrolidino, piperidino, piperazino, 4-alkylpiperazino, morpholino and so on. These substituents are one, or two or more independently from each other.

Examples of the "cycloalkyl group" include a cycloalkyl group having 8 or less carbon atoms, such as cyclopentyl, cyclohexyl and so on.

Preferable examples of $R^2$ and $R^3$ for the above-mentioned formula include an optionally substituted alkyl group. More preferable is an alkyl group, especially methyl and ethyl.

Preferable examples of $R^1$ and $R^6$ for the above-mentioned formula are hydrogen atom.

Preferable examples of n for the above-mentioned formula are 0 and 1, especially 0.

When the prodrug of the amide given by the formula 1 or the prodrug given by the formula 2 or 3 has a basic or acidic group, it can be derived to a salt with an acid or base according to a conventional method.

Examples of the pharmaceutically acceptable salt include salts with inorganic acids or organic acids. Examples of the inorganic acid include hydrochloric acid, hydrobromic acid, hydroiodic acid, sulfuric acid and so on. Examples of the organic acid include acetic acid, oxalic acid, citric acid, malic acid, tartaric acid, maleic acid, fumaric acid and so on.

Examples of the pharmaceutically acceptable salt include salts with inorganic bases or organic bases. Examples of the inorganic base include sodium hydroxide, potassium hydroxide, calcium hydroxide and so on. Examples of the organic base include basic amino acid such as arginine, lysine and so on.

The prodrug of the amide given by the formula 1 or the prodrug given by the formula 2 or 3, and pharmaceutically acceptable salt thereof may be solvate such as hydrate and so on. Further, when there are tautomers, geometrical isomers or stereoisomers, the present invention includes a mixture of each of the isomers, and isolated isomer.

The prodrug of the present invention can be produced by conventional methods in the field of organic synthetic chemistry. For example, the compounds modified with a typical functional group such as acyl group, optionally substituted phosphono group and optionally substituted alkanoyloxymethyl group can be produced as follows.

Namely, the prodrugs given by formula 2:

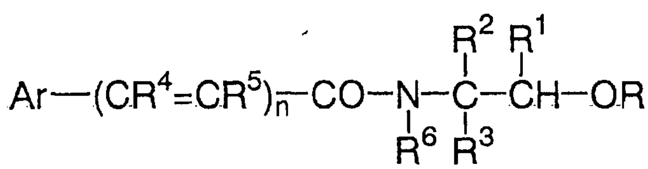

(2)

can be produced by modifying the amide given by formula 1:

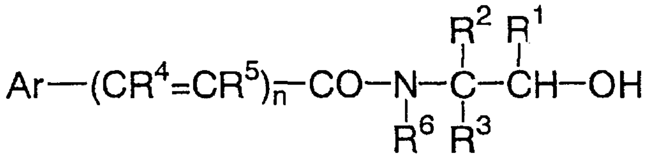

(1)

wherein $R^1$, $R^2$, $R^3$, $R^4$, $R^5$, $R^6$, R and Ar are as defined above,
with an R group.

The acylation reaction can be usually carried out according to general methods when R is an acyl group. For example, a prodrug, whose R is an acyl group, can be produced by condensing a carboxylic acid (R-OH), protected an active functional group by a suitable protective group if necessary, with an amide given by the formula 1 by using a condensation agent such as mixture of WSC hydrochloride and 4-dimethylaminopyridine, and performing deprotection.

A compound, whose R is an optionally substituted phosphono group, can be produced by making an amide given by the formula 1 react with a halophosphate according to conventional methods. For example, trialkyl phosphate can be produced by making the amide given by the formula 1 react with a dialkyl chlorophosphate. Further, monoalkyl phosphate can be produced by making the amide react with dibenzyl chlorophosphate, and then performing a debenzylation by catalytic reduction and the like.

A compound, whose R is an optionally substituted alkanoyloxymethyl group, can be produced by carrying out a methylthiomethylation (cf. "Protective Groups in Organic Synthesis" T. W. Greene, P. M. Wuts John. Wiley and sons 1991, pp. 21-22, etc.) for hydroxy group of an amide given by the formula 1, and then introducing R group by a reaction with a carboxylic anhydride (optionally substituted alkanoic anhydride) according to conventional methods. For example, a prodrug whose R is acetoxymethyl group can be produced by making a methylthiomethylated compound react with acetic anhydride (J. Chem. Research (S), 68 (1992)). Further, a compound, whose R is a substituted alkanoyloxymethyl group, can be produced by a conversion to chloroacetoxymethyl group by a known method (e.g. JP 8-225558(A)) and then a conversion to "substituent" such as amino group from chlorine atom, if necessary, according to conventional methods

Furthermore, a prodrug given by the formula 2 or 3 and an amide given by the formula 1 above can be produced as follows:

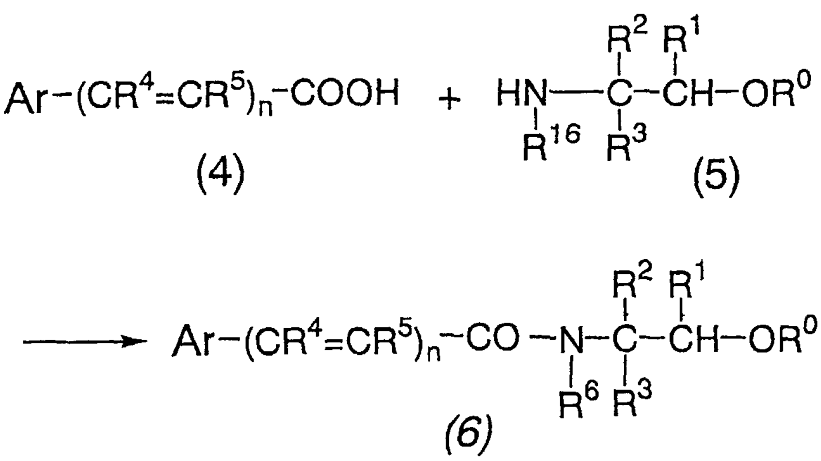

(4) (5)

(6)

wherein $R^0$ represents the same meaning as R above, or a hydrogen atom. $R^{16}$ represented the same meaning as $R^6$ above, or a protected hydroxy group. Ar, n, $R^1$, $R^2$, $R^3$, $R^4$, $R^5$ and $R^6$ mean as defined above.

Namely, a compound of the formula 6 can be produced by making a carboxylic acid given by the formula 4 condense with an amine given by the formula 5, and, when $R^{16}$ is a protected hydroxy group, performing deprotection according to conventional methods. The compound of the formula 6 corresponds to a prodrug of the above-formula 2 when R° is R, and corresponds to a prodrug of the formula 1 above when $R^0$ is a hydrogen atom. The above starting compound 5, whose $R^0$ is R, can be produced by modifying hydroxy group of a compound of the formula 5 similarly to the method for producing the amide of the above formula 2, after protecting amino group with a suitable protecting group if necessary, and performing deprotection.

Protective groups for hydroxy group of $R^{16}$ include protective groups conventionally used in the field of organic

synthetic chemistry (cf. "Protective Groups in Organic Synthesis" T. W. Greene, P. M. Wuts John. Wiley and sons 1991, pp. 10-142, etc.). Typical examples are substituted silyl group such as trimethylsilyl, triisopropylsilyl, dimethylisopropylsilyl, diethylisopropylsilyl, t-butyldimethylsilyl, diphenylmethylsilyl, t-butyldiphenylsilyl and the like; optionally substituted methyl group such as t-butyl, benzyl, trityl, methoxymethyl, methylthiomethyl, benzyloxymethyl, methoxyethoxymethyl, tetrahydropyranyl and the like, and the like. Preferable are t-butyl, benzyl, trityl, methoxymethyl, tetrahydropyranyl, benzyloxymethyl, methoxyethoxymethyl and the like.

The condensation reaction of the carboxylic acid of the formula 4 with the amine of the formula 5 can be carried out according to conventional methods in the field of peptide chemistry (cf. "Basis and Experiment in Peptide Synthesis" by Nobuo Izumiya et.al., Maruzen, etc.). Typical methods are C-terminal activation methods (e.g. acid halide methods, acid azide methods, mixed acid anhydride methods, activated ester methods, symmetrical acid anhydride methods and the like), methods using coupling agents (methods using N,N'-dicyclohexylcarbodiimide and the like), N-terminal activation methods (isocyanate methods, phosphazo methods, phosphorous ester methods and the like) and the like.

The acid halide methods are carried out, for example, by converting a carboxylic acid of the formula 4 to acid halide according to conventional methods, followed by condensing with a compound of the formula 5 at 0°C to room temperature in the presence of a base in an inert solvent such as dichloromethane and so on. Examples of the base include organic base such as triethylamine and so on.

The methods using coupling agents are carried out, for example, by condensing a carboxylic acid of the formula 4 with an amine of the formula 5 in the presence of a coupling agent such as N-(3-dimethylaminopropyl)-N'-ethylcarbodiimide hydrochloride (WSC hydrochloride), together with 1-hydroxybenzotriazole (HOBt) if necessary, at 0°C to room temperature in an inert solvent such as dichloromethane, N,N-dimethylformamide (DMF) and so on. When Ar is optionally substituted pyridyl group, N-oxide compound can be produced by making the carboxylic acid of the formula 4 react with the amine of the formula 5 and then oxidizing the product with a suitable oxidizing agent. For example, it may be oxidized with oxidizing agent such as aqueous hydrogen peroxide and so on in a solvent such as acetic acid, trifluoroacetic acid and so on at room temperature to refluxing temperature.

When $R^{16}$ is a protected hydroxy group, the protecting group of hydroxy group can be removed by conventional methods. For example, hydrogenolysis or hydrolysis using acid catalyst can remove the protecting group when the protecting group is t-butyl, benzyl, trityl, methoxymethyl, tetrahydropyranyl, benzyloxymethyl, methoxyethoxymethyl and so on.

The compounds obtained by the above methods can be purified by conventional methods. For example, they may be purified by column chromatography, recrystallization and so on. Examples of recrystallization solvent include alcohols such as methanol, ethanol, 2-propanol and the like; ethers such as diethyl ether and the like; esters such as ethyl acetate and the like; aromatic solvents such as toluene and the like; ketones such as acetone and the like; hydrocarbons such as hexane and the like; water; mixtures thereof and so on.

The medicament for treating retinal neurodegenerative disorders of the present invention may be administered orally or parenterally (e.g. intramuscular injection, intravenous injection, rectal administration by suppository, dermal application for liniments, eye drops or the like). Pharmaceutical forms for oral administration include, for example, tablets, capsules, syrups, suspensions and the like. Pharmaceutical forms for injection include, for example, solutions, emulsions, suspensions and the like. These compositions can be prepared by mixing the active ingredient with conventional carriers, excipients, binders, stabilizers and the like by conventional methods. Injection may contain buffers, solubilizers, isotonic agents and the like. Though the dose and the frequency for administration varies depending on the grade of the symptoms, the patient's age, body weight, administration route and the like, the active ingredient is usually administered to an adult in a dose of 1 - 1000 mg, preferably 10 - 500 mg per day in one portion or several portions, by the oral route. By injection, the active ingredient is usually administered in a dose of 0.1 - 500 mg, preferably 3 - 100 mg in one portion or several portions.

The medicament of the present invention is, for example, useful for treating retinal neurodegenerative disorders such as retinitis pigmentosa, senile macular degeneration, diabetic retinopathy, glaucoma, traumatic retinodialysis and so on. Further, the medicament of the present invention is also useful for treating neurodegenerative disorders such as cerebral apoplexy, hypoglycemia, cardiac arrest, perinatal asphyxia, neurocyte degenerative disorders caused by medulla spinalis trauma and the like, epilepsy, Huntington's chorea, Parkinson's disease, Alzheimer's disease, diabetic neurodegenerative disorders and so on.

## Examples

The present invention will be described in detail below, referring to examples, which are not limitative of the present invention.

The following typical compounds are shown as prodrugs of the present invention:

N-(2-acetoxy-1,1-dimethylethyl)-2,4-difluorobenzamide,
N-(2-propionyloxy-1,1-dimethylethyl)-2,4-difluorobenzamide,
N-(2-butyryloxy-1,1-dimethylethyl)-2,4-difluorobenzamide,
N-(2-isobutyryloxy-1,1-dimethylethyl)-2,4-difluorobenzamide,
N-(2-valeryloxy-1,1-dimethylethyl)-2,4-difluorobenzamide,
N-(2-isovaleryloxy-1,1-dimethylethyl)-2,4-difluorobenzamide,
N-(2-pivaloyloxy-1,1-dimethylethyl)-2,4-difluorobenzamide,
N-(2-lauroyloxy-1,1-dimethylethyl)-2,4-difluorobenzamide,
N-(2-myristoyloxy-1,1-dimethylethyl)-2,4-difluorobenzamide,
N-(2-palmitoyloxy-1,1-dimethylethyl)-2,4-difluorobenzamide,
N-(2-stearoyloxy-1,1-dimethylethyl)-2,4-difluorobenzamide,
N-(2-benzoyloxy-1,1-dimethylethyl)-2,4-difluorobenzamide,
N-(2-(2-carboxybenzoyloxy)-1,1-dimethylethyl)-2,4-difluorobenzamide,
N-(2-(2-aminobenzoyloxy)-1,1-dimethylethyl)-2,4-difluorobenzamide,
N-(2-(2-hydroxybenzoyloxy)-1,1-dimethylethyl)-2,4-difluorobenzamide,
N-(2-(3-carboxypropanoyloxy)-1,1-dimethylethyl)-2,4-difluorobenzamide,
N-(2-glycyloxy-1,1-dimethylethyl)-2,4-difluorobenzamide,
N-(2-β-alanyloxy-1,1-dimethylethyl)-2,4-difluorobenzamide,
N-(2-methoxycarbonyloxy-1,1-dimethylethyl)-2,4-difluorobenzamide,
N-(2-t-butoxycarbonyloxy-1,1-dimethylethyl)-2,4-difluorobenzamide,
N-(2-aminocarbonyloxy-1,1-dimethylethyl)-2,4-difluorobenzamide,
N-(2-phosphonooxy-1,1-dimethylethyl)-2,4-difluorobenzamide,
N-(2-acetoxy-1,1-dimethylethyl)-5-chloro-2-pyridinecarboxamide,
N-(2-propionyloxy-1,1-dimethylethyl)-5-chloro-2-pyridinecarboxamide,
N-(2-pivaloyloxy-1,1-dimethylethyl)-5-chloro-2-pyridinecarboxamide,
N-(2-benzoyloxy-1,1-dimethylethyl)-5-chloro-2-pyridinecarboxamide,
N-(2-palmitoyloxy-1,1-dimethylethyl)-5-chloro-2-pyridinecarboxamide,
N-(2 - (3-carboxypropionyloxy)-1,1-dimethylethyl)-5-chloro-2-pyridinecarboxamide,
N-(2-glycyloxy-1,1-dimethylethyl)-5-chloro-2-pyridinecarboxamide,
N-(2-acetoxy-1,1-dimethylethyl)-5-chloro-1-oxide-2-pyridinecarboxamide and
N-(2-pivaloyloxy-1,1-dimethylethyl)-5-chloro-1-oxide-2-pyridinecarboxamide,

Example 1

<u>N-(2-Acetoxy-1,1-dimethylethyl)-2,4-difluorobenzamide</u>

1) N-(2-Hydroxy-1,1-dimethylethyl)-2,4-difluorobenzamide

To a mixture of 2,4-difluorobenzoic acid (6.32 g, 40 mmol), 2-amino-2-methyl-1-propanol (3.56 g, 40 mmol), HOBt (5.40 g, 40 mmol) and dichloromethane (150 ml), WSC hydrochloride (7.68 g, 40 mmol) was added and stirred for 3 hours at room temperature. The concentrated reaction solution was diluted with ethyl acetate, washed with saturated aqueous sodium bicarbonate, IN hydrochloric acid, saturated aqueous sodium bicarbonate and saturated brine subsequently, dried over magnesium sulfate and concentrated under reduced pressure. The residue was purified by silica gel chromatography (hexane / ethyl acetate = 3 / 1) to give the title compound (6.20 g; 68%).
$^1$H-NMR(270 MHz, $CDCl_3$) δ 8.08 (td, J=8.8, 6.6 Hz, 1H), 6.96-7.04 (m, 1H), 6.87 (ddd, J=12, 8, 2 Hz, 1H), 6.76 (br, 1H), 4.43 (t, J=6 Hz, 1H), 3.69 (d, J=6 Hz, 2H), 1.42 (s, 6H)

2) N-(2-Acetoxy-1,1-dimethylethyl)-2,4-difluorobenzamide

To a solution of N-(2-hydroxy-1,1-dimethylethyl)-2,4-difluorobenzamide (458 mg, 2 mmol), 4-dimethylaminopyridine (366 mg, 3 mmol) in dichloromethane (5 ml), acetic anhydride (306 mg, 3 mmol) was added under ice-cooling and stirred at the temperature for 30 minutes, and further stirred overnight at room temperature. Dichloromethane was evaporated under reduced pressure and the residue was diluted with ethyl acetate. The dilution was washed with IN hydrochloric acid, aqueous saturated sodium bicarbonate and saturated brine subsequently, dried over magnesium sulfate and concentrated under reduced pressure. The residue was purified by silica gel chromatography (hexane / ethyl acetate = 5 / 1) to give the title compound (520 mg; 96%).
$^1$H-NMR(270 MHz, $CDCl_3$) δ 8.06 (td, J=8.9, 6.6 Hz, 1H), 6.95-7.02 (m, 1H), 6.85 (ddd, J=12, 8, 2 Hz, 1H), 6.65 (br, 1H), 4.28 (s, 2H), 2.10 (s, 3H), 1.48 (s, 6H)

Example 2

N-(2-Benzoyloxy-1,1-dimethylethyl)-2,4-difluorobenzamide

To a solution of N-(2-hydroxy-1,1-dimethylethyl)-2,4-difluorobenzamide obtained in Example 1-1 (458 mg, 2 mmol), triethylamine (303 mg, 3 mmol), 4-dimethylaminopyridine (24 mg, 0.2 mmol) in dichloromethane (6 ml), benzoic anhydride (678 mg, 3 mmol) was added and stirred for 3 hours at room temperature and heated under reflux for 1 hour. Dichloromethane was evaporated under reduced pressure and the residue was diluted with ethyl acetate. The dilution was washed with water, 1N hydrochloric acid, aqueous saturated sodium bicarbonate and saturated brine subsequently, dried over sodium sulfate and concentrated under reduced pressure. The residue was purified by silica gel chromatography (hexane / ethyl acetate = 10 / 1) to give the title compound (638 mg; 96%).
$^1$H-NMR(270 MHz, $CDCl_3$) δ 8.03-8.12 (m, 3H), 7.57 (t, J=7.2 Hz, 1H), 7.44 (t, J= 8 Hz, 2H), 6.94-7.02 (m, 1H), 6.85 (ddd, J=11, 8, 2 Hz, 1H), 6.75 (br, 1H), 4.53 (s, 2H), 1.58 (s, 6H)

Example 3

N-(2-(3-Carboxypropionyloxy)-1,1-dimethylethyl)-2,4-difluorobenzamide

A mixture of N-(2-hydroxy-1,1-dimethylethyl)-2,4-difluorobenzamide obtained in Example 1-1 (6.87 g, 30 mmol), succinic anhydride (3.3 g, 33 mmol), sodium carbonate (3.50 g, 33 mmol) and toluene (100 ml) was heated under reflux for 5 hours. After cooling, the reaction solution was poured into water (100 ml), made acidic with IN hydrochloric acid, and then extracted with ethyl acetate twice. The combined extracts were dried over sodium sulfate and concentrated under reduced pressure. The residue was purified by silica gel chromatography (chloroform / methanol = 50 / 1) and recrystallized from hexane / chloroform to give the title compound (3.65 g; 37%).
$^1$H-NMR(270 MHz, $CDCl_3$) δ 8.04 (td, J=8.9, 6.6 Hz, 1H), 6.94-7.01 (m, 1H), 6.84 (ddd, J=12, 8, 2 Hz, 1H), 6.60 (br, 1H), 5.5 (br, 1H), 4.33 (s, 2H), 2.66 (s, 4H), 1.47 (s, 6H)

Example 4

N-(2-Glycyloxy-1,1-dimethylethyl)-2,4-difluorobenzamide benzenesulfonate

1) N-(2-(t-Butyloxycarbonylglycyloxy)-1,1-dimethylethyl)-2,4-difluorobenzamide

To a solution of N-(2-hydroxy-1,1-dimethylethyl)-2,4-difluorobenzamide obtained in Example 1-1 (4.58 g, 20 mmol), N-t-butyloxycarbonylglycine (3.85 g, 22 mmol), 4-dimethylaminopyridine (0.37 g, 6 mmol) in DMF (50 ml), WSC hydrochloride (4.22 g, 22 mmol) was added and stirred at room temperature for 2 hours. The reaction solution was diluted with ethyl acetate (200 ml), washed with 5% aqueous potassium hydrogensulfate, aqueous saturated sodium bicarbonate and saturated brine subsequently, and dried over sodium sulfate. The solvent was evaporated under reduced pressure to give the title compound (7.7 g; 99%).
$^1$H-NMR(270 MHz, $CDCl_3$) δ 8.05 (td, J=8.9, 6.6 Hz, 1H), 6.95-7.02 (m, 1H), 6.85 (ddd, J=12, 8, 2 Hz, 1H), 6.55 (br, 1H), 5.01 (br, 1H), 4.40 (s, 2H), 3.94 (d, J=6Hz, 2H), 1.47 (s, 6H), 1.43 (s, 9H)

2) N-(2-Glycyloxy-1,1-dimethylethyl)-2,4-difluorobenzamide benzenesulfonate

To a solution of N-(2-(t-butyloxycarbonylglycyloxy)-1,1-dimethylethyl)-2,4-difluorobenzamide (1.16 g, 3 mmol) in acetonitrile (12 ml), benzenesulfonic acid hydrate (1.06 g, 6 mmol) was added and stirred at room temperature for-3 days. The precipitate was collected by filtration and dried to give the title compound (0.94 g; 70%).
$^1$H-NMR(270 MHz, DMSO-$d_6$) δ 8.19 (br, 3H), 7.95 (s, 1H), 7.56-7.65 (m, 3H), 7.27-7.35 (m, 4H), 7.14 (t, J=8 Hz, 1H), 4.40 (s, 2H), 3.86 (br, 2H), 1.36 (s. 6H)

Example 5

N-(2-(2-Carboxybenzoyloxy)-1,1-dimethylethyl)-5-chloro-2-pyridinecarboxamide

1) 5-Chloro-2-vinylpyridine

To a solution of tributyl(vinyl)tin (6.33 g, 20.0 mmol), 2,5-dichloropyridine (2.96 g, 20.0 mmol) in toluene (30 ml), tetrakis (triphenylphosphine)palladium(0) (0.46 g, 0.4 mmol) was added and heated under reflux for 3 hours. To

aqueous ammonium fluoride solution (100 ml), the reaction mixture and ethyl acetate (30 ml) were added subsequently, and stirred for 1 hour. The white precipitate was filtered off and the filtrate was collected. The aqueous layer was extracted with ethyl acetate three times and the combined organic layers were dried over sodium sulfate. The solution was subjected to purification by silica gel chromatography (hexane / ethyl acetate = 10 / 1 - 2 / 1), and the eluent was concentrated under atmospheric pressure to give the oil containing the title compound (3.6 g).
$^1$H-NMR(270 MHz, $CDCl_3$) δ 8.52 (d, J=2.4 Hz, 1H), 7.62 (dd, J=8.5, 2.4 Hz, 1H), 7.29 (d, J=8.5 Hz, 1H), 6.78 (dd, J=17.5, 10.7 Hz, 1H), 6.18 (dd, J=17.5, 1.3 Hz, 1H), 5.51 (dd, J=10.7, 1.3 Hz, 1H)

2) 5-Chloro-2-pyridinecarboxylic acid

To a solution of sodium periodate (2.13 g, 9.98 mmol), potassium permanganate (0.065 g, 0.41 mmol) in water (10 ml), potassium carbonate (0.29 g, 2.1 mmol) was added gradually. After adding t-butanol (5 ml), 5-chloro-2-vinylpyridine (0.34 g, approximately 2 mmol) obtained above was added gradually and stirred for 1 hour. Ethylene glycol (1 ml) was added thereto and further stirred for 1 hour. The reaction mixture was added to aqueous 5% potassium hydrogensulfate, extracted with ethyl acetate three times and dried over sodium sulfate. The solvent was evaporated to give the title compound (0.31 g).
$^1$H-NMR(270 MHz, DMSO-$d_6$) δ 8.76 (dd, J=2.3, 0.5 Hz, 1H), 8.12 (dd, J=8.3, 2.3 Hz, 1H), 8.04 (dd, J=8.3, 0.5 Hz, 1H)

3) N-(2-hydroxy-1,1-dimethylethyl)-5-chloro-2-pyridinecarboxamide

It was obtained by the reaction of 5-chloro-2-pyridinecarboxylic acid with 2-amino-2-methyl-1-propanol similar to procedure of Example 1-1.
$^1$H-NMR(270 MHz, $CDCl_3$) δ 8.49 (dd, J=2.4, 0.7 Hz, 1H), 8.14 (dd, J=8.6, 0.7 Hz, 1H), 8.05 (br, 1H), 7.83 (dd, J=8.6, 2.4 Hz, 1H), 4.70 (t, J=6.4 Hz, 1H), 3.73 (d, J=6.4 Hz, 2H), 1.43 (s, 6H)

4) N-(2-(2-carboxybenzoyloxy)-1,1-dimethylethyl)-5-chloro-2-pyridinecarboxamide

A mixture of N-(2-hydroxy-1,1-dimethylethyl)-5-chloro-2-pyridinecarboxamide (458 mg, 2 mmol), phthalic anhydride (326 mg, 2.2 mmol), sodium carbonate (233 mg, 2.2 mmol), 4-dimethylaminopyridine (24 mg, 0.2 mmol) and toluene (5 ml) was heated under reflux for 5 hours. The reaction mixture was allowed to cool, poured into IN hydrochloric acid and extracted with ethyl acetate twice. The combined extracts were dried over sodium sulfate and concentrated under reduced pressure. The residue was purified by silica gel chromatography (chloroform / methanol = 50 / 1), and recrystallized from hexane / chloroform to give the title compound (422 mg; 56%).
$^1$H-NMR(270 MHz, $CDCl_3$) δ 8.54 (d, J=2.3 Hz, 1H), 8.16 (d, J=8.6 Hz, 1H), 7.89 (dd, J=8.6, 2.3 Hz, 1H), 7.84-7.87 (m, 1H), 7.71-7.74 (m, 1H), 7.65 (br, 1H), 7.54-7.62 (2H, m), 4.6 (br, 1H), 4.44 (s, 2H), 1.57 (s, 6H)
The structures of the compounds obtained in Examples 1 to 5 are as follows:

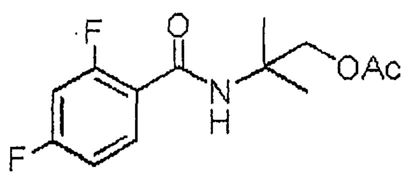

Example 1

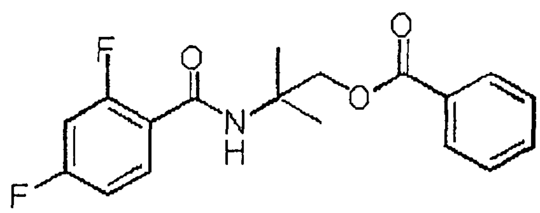

Example 2

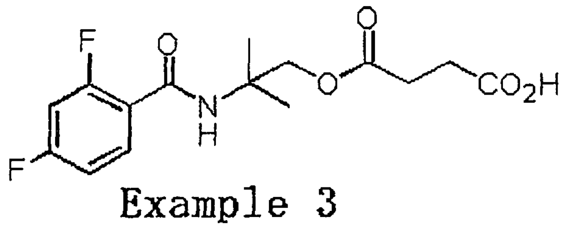

Example 3

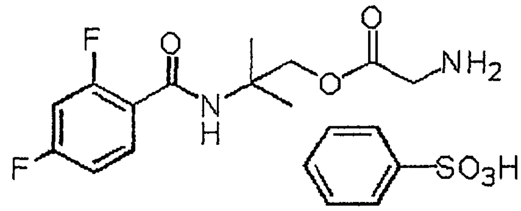

Example 4

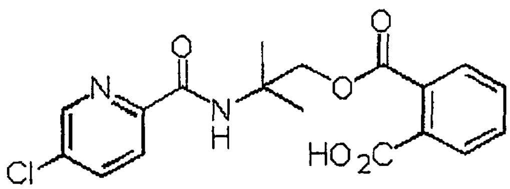

Example 5

Retinal outer nuclear cells have been known to be degenerated by the constant white light exposure to albino rats (L. M. Rapp, et al., New York: Plenum, 135 (1980)). Effectiveness of the prodrugs of the present invention or pharmaceutical acceptable salts thereof can be evaluated by their protective action against constant light exposure-induced retinal degeneration in rats. Effectiveness of the present invention is embodied by the following experiment.

Experiment 1

Pharmacological effect against constant white light-induced retinal damage

Male Wistar rats (7 weeks old, Charles River Japan Inc.) were purchased and maintained in cyclic light / dark environment (8:00-20:00 / light term) for one week, and then placed within the apparatus for constant white light exposure for two days. The constant white light exposure apparatus is a covered breeding box of 1020 mm in length, 425 mm in width, 520 mm in height, which is made of acryl boards and of which all inner side are covered with mirrors. Light was continuously irradiated all the day long (24 hours) using white fluorescent lamp attached to the ceiling of the apparatus. The average illuminance in the apparatus was 174.2 foot candle. After two days in the apparatus, the rats were moved to a darkroom and dark adapted for 1-4 hours. The rats were anesthetized with pentobarbital and placed in a stereotaxic frame. After topical mydriatica was given and electrodes were put on corneal surface, center of the forehead and the lower part of the lobe, the response in active potential of retina to the flash stimulation with fixed energy was determined from the recorded ERG (electroretinogram). The damage of retina was evaluated by the decrease in the amplitude of ERG a-wave which originated from retinal outer nuclear cells (photoreceptors). Test compounds were intraperitoneally injected just before placing the rats into the constant light exposure apparatus and at the same time on the next day to investigate its protective effect.

According to the experimental procedure described above, each test compound of the present invention, which was suspended or dissolved in 0.5 % methylcellulose (MC) solution, was orally administered five rats in a group at the dose of 100 mg/kg. In the same way, 0.5 % MC solution was orally administered to the rats in MC group. The rats maintained in 12 hours cyclic light / dark environment were used for normal control group. The recovery degree against the damage was represented as % recovery value, and the results were shown in Table 1.

$$\% \text{ recovery} = (x - z) \div (y - z) \times 100$$

x : a-wave amplitude in test compound group
y : a-wave amplitude in normal control group
z : a-wave amplitude in MC group

Table 1 :

| Pharmacological effect of tested compounds | | |
|---|---|---|
| Test Compounds | % recovery (Mean±S.E.M.) | N |
| The compound of Example 3 | 14.0 ± 3.1 | 5 |
| The compound of Example 4 | 19.5 ± 3.3 | 5 |

## Claims

**1.** A prodrug of an amide given by formula 1:

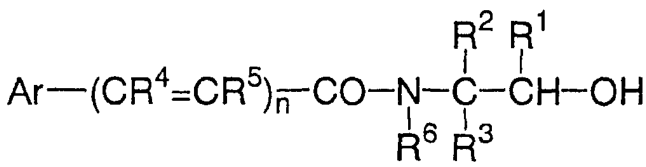

wherein Ar is an optionally substituted phenyl group or optionally substituted aromatic heterocyclic group; n is an integer of 0, 1 or 2;

$R^1$ is a hydogen atom or an optionally substituted alkyl group; $R^2$ and $R^3$ are independently an optionally substituted alkyl group; or $R^2$ may be bonded with
$R^1$ or $R^3$ and taken together with the carbon atom adjacent thereto to form an optionally substituted cycloalkane ring;
$R^4$ and $R^5$ are independently a hydrogen atom or an optionally substituted alkyl group;
$R^6$ is a hydrogen atom, a hydroxy group or an alkyl group;
or a pharmaceutically acceptable salt thereof.

**2.** A compound described in claim 1, which is a prodrug given by formula:

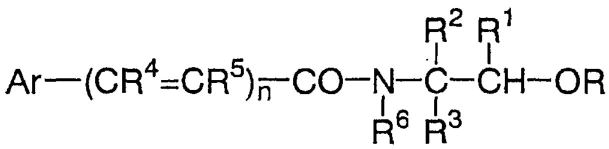

wherein Ar, n, $R^1$, $R^2$, $R^3$, $R^4$, $R^5$ and $R^6$ are as defined in claim 1; and R is an acyl group, optionally substituted phosphono group or optionally substituted alkanoyloxymethyl group;
or a pharmaceutically acceptable salt thereof.

**3.** A compound described in claim 2, wherein the acyl group is an optionally substituted alkanoyl group, optionally substituted aroyl group, optionally substituted alkoxycarbonyl group or optionally substituted aminocarbonyl group.

**4.** A compound described in any one of claims 1-3, wherein n is 0.

**5.** A compound described in any one of claims 1-4, wherein the substituents for substituted phenyl or substituted aromatic heterocyclic group are halogen atom, cyano group, nitro group, alkyl group, halogen-substituted alkyl group, alkoxy group, halogen-substituted alkoxy group, alkoxycarbonyl group, alkanoylamino group, amino group, phenyl group, alkylaminocarbonylamino group, alkoxycarbonylamino group, alkylsulfonylamino group, carbamoyl group or alkyl-substituted carbamoyl group; and the substituents for substituted alkyl group or substituted cycloalkane ring are, independently from each other, cycloalkyl group, alkoxy group, hydroxy group, halogen atom, alkoxyalkoxy group, alkanoyloxy group, amino group, alkylamino group, dialkylamino group, alkanoylamino group, pyrrolidino group, piperidino group, piperazino group, 4-alkylpiperazino group or morpholino group.

**6.** A compound described in any one of claims 1-4, wherein Ar is a phenyl group, 2-pyridyl group, 3-pyridyl group, 4-pyridyl group, pyrazinyl group, 3-pyridazinyl group, 4-pyridazinyl group, 2-pyrimidinyl group, 4-pyrimidinyl group or 5-pyrimidinyl group (said Ar group may be substituted by 1-3 halogen atoms, and the nitrogen atom in said 2-pyridyl, 3-pyridyl and 4-pyridyl group may be oxidized).

**7.** A compound described in any one of claims 1-4, wherein Ar is a 2,4-difluorophenyl group, 2,4-dichlorophenyl group, 4-fluorophenyl group, 4-chlorophenyl group, 2-fluoro-4-chlorophenyl group, 2-fluoro-4-bromophenyl group, 2-fluoro-4-iodophenyl group, 2,4,5-trifluorophenyl group, 5-chloro-2-pyridyl group, 5-fluoro-2-pyridyl group, 3-fluoro-2-pyridyl group, 2-chloro-5-pyridyl group, 1-oxide-2-pyridyl group, 1-oxide-5-chloro-2-pyridyl group, 1-oxide-5-fluoro-2-pyridyl group, 1-oxide-3-fluoro-2-pyridyl group, 1-oxide-2-chloro-5-pyridyl group or 6-chloro-3-pyridazinyl group.

**8.** A compound described in any one of claims 1-7, wherein $R^1$ is a hydrogen atom.

**9.** A compound described in any one of claims 1-8, wherein $R^6$ is a hydrogen atom.

**10.** A medicament for treating neurodegenerative disorders, comprising a compound described in any one of claims 1-9.

**11.** A medicament comprising a prodrug given by formula:

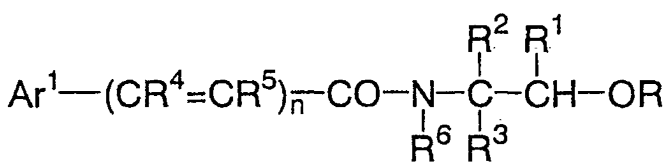

wherein $Ar^1$ is a phenyl substituted by fluorine atom at 2- or 4-position (said phenyl may be further substituted with one or two halogen atoms) or optionally substituted 6-membered aromatic heterocyclic group; R, n, $R^1$, $R^2$, $R^3$, $R^4$, $R^5$ and $R^6$ are as defined in claim 2;
or a pharmaceutically acceptable salt thereof.

**12.** A medicament described in claim 11, wherein n is 0.

**13.** A medicament described in claim 11 or 12, wherein the substituents for the substituted 6-membered aromatic heterocyclic group are halogen atom, cyano group, nitro group, alkyl group, halogen-substituted alkyl group, alkoxy group, halogen-substituted alkoxy group, alkoxycarbonyl group, alkanoylamino group, amino group, phenyl group, alkylaminocarbonylamino group, alkoxycarbonylamino group, alkylsulfonylamino group, carbamoyl group or alkyl-substituted carbamoyl group; and the substituents for the substituted alkyl group or substituted cycloalkane ring are, independently from each other, cycloalkyl group, alkoxy group, hydroxy group, halogen atom, alkoxyalkoxy group, alkanoyloxy group, amino group, alkylamino group, dialkylamino group, alkanoylamino group, pyrrolidino group, piperidino group, piperazino group, 4-alkylpiperazino group or morpholino group.

**14.** A medicament described in claim 11 or 12, wherein the "substituents" for the "optionally substituted 6-membered aromatic heterocyclic group" are 1-3 halogen atoms; and the "6-membered aromatic heterocyclic group" is a 2-pyridyl group, 3-pyridyl group, 4-pyridyl group, pyrazinyl group, 3-pyridazinyl group, 4-pyridazinyl group, 2-pyrimidinyl group, 4-pyrimidinyl group or 5-pyrimidinyl group (the nitrogen atom in said 2-pyridyl, 3-pyridyl and 4-pyridyl group may be oxidized).

**15.** A medicament described in claim 11 or 12, wherein $Ar^1$ is a 2,4-difluorophenyl group, 4-fluorophenyl group, 2-fluoro-4-chlorophenyl group, 2-fluoro-4-bromophenyl group, 2-fluoro-4-iodophenyl group, 2,4,5-trifluorophenyl group, 5-chloro-2-pyridyl group, 5-fluoro-2-pyridyl group, 3-fluoro-2-pyridyl group, 2-chloro-5-pyridyl group, 1-oxide-2-pyridyl group, 1-oxide-5-chloro-2-pyridyl group, 1-oxide-5-fluoro-2-pyridyl group, 1-oxide-3-fluoro-2-pyridyl group, 1-oxide-2-chloro-5-pyridyl group or 6-chloro-3-pyridazinyl group.

**16.** A medicament described in any one of claims 11-15, wherein $R^1$ is a hydrogen atom, and each of $R^2$ and $R^3$ is a

methyl group.

**17.** A medicament described in any one of claims 11-16, wherein $R^6$ is a hydrogen atom.

**18.** A prodrug given by formula:

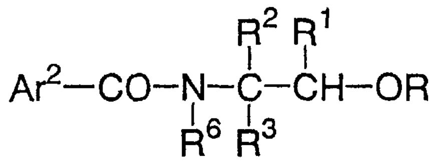

wherein $Ar^2$ is a group of formula:

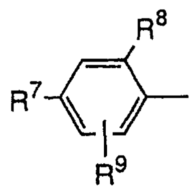

or a 6-membered aromatic heterocyclic group substituted with 1-3 halogen atoms; R, n, $R^1$, $R^2$, $R^3$ and $R^6$ are as defined in claim 2; one of $R^7$ and $R^8$ is a fluorine atom and the other is a hydrogen atom or a halogen atom; and $R^9$ is a hydrogen atom or a halogen atom; or a pharmaceutically acceptable salt thereof.

**19.** A compound described in claim 18, wherein the 6-membered aromatic heterocyclic group is a pyrazinyl, 3-pyridazinyl, 4-pyridazinyl, 2-pyrimidinyl, 4-pyrimidinyl, 5-pyrimidinyl, 2-pyridyl, 3-pyridyl, 4-pyridyl, 1-oxide-2-pyridyl, 1-oxide-3-pyridyl or 1-oxide-4-pyridyl group

**20.** A compound described in claim 18, wherein $Ar^2$ is a 2,4-difluorophenyl, 2-fluoro-4-chlorophenyl, 2-fluoro-4-bromophenyl, 2-fluoro-4-iodophenyl, 2,4,5-trifluorophenyl, 5-chloro-2-pyridyl, 5-fluoro-2-pyridyl, 3-fluoro-2-pyridyl, 2-chloro-5-pyridyl, 1-oxide-2-pyridyl group, 1-oxide-5-chloro-2-pyridyl, 1-oxide-5-fluoro-2-pyridyl, 1-oxide-3-fluoro-2-pyridyl, 1-oxide-2-chloro-5-pyridyl or 6-chloro-3-pyridazinyl group.

**21.** A compound described in any of claims 18-20, wherein $R^1$ is a hydrogen atom, and $R^2$ and $R^3$ are methyl groups.

**22.** A compound described in any of claims 18-21, wherein $R^6$ is a hydrogen atom.

## INTERNATIONAL SEARCH REPORT

International application No.
PCT/JP00/02470

**A. CLASSIFICATION OF SUBJECT MATTER**

Int.Cl[7] C07C237/32, C07D213/81, A61K31/216, A61K31/222, A61K31/44, A61K31/166, A61P25/28, A61P25/00, A61P25/16, A61P25/14, A61P25/08, A61P27/06, A61P27/08, C07D237/24, C07D239/28

According to International Patent Classification (IPC) or to both national classification and IPC

**B. FIELDS SEARCHED**

Minimum documentation searched (classification system followed by classification symbols)
Int.Cl[7] C07C, C07D, A61K, A61P

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| PX | WO, 99/21543, A1 (Sumitomo Pharmaceuticals Co. Ltd.), 06 May, 1999 (06.05.99), Full text; especially see Claims; Abstract; page 8, lines 6 to 12; Examples 27, 33, 47; page 34, lines 3 to 9; page 34, line 10 to page 35, line 24 (Family: none) | 1-22 |
| X | JP, 9-176125, A (Nissan Chemical Industries Ltd.), 08 July, 1997 (08.07.97), page 30, lines 1 to 2, (Compounds No.2-337, 2-338), & WO, 96/38419, A1 | 1-5,8,9 |
| X | JP, 58-52343, A (Asahi Chemical Industry Co., Ltd.), 28 March, 1983 (28.03.83), page 7 (Family: none) | 1-6,8,9 |
| X | MORCUENDE Anabel, et al., "Microwave-Promoted Transformation: Fast and Chemoselective N-Acylation of Amino Alcohols Using Catalytic Amounts of Dibutyltin Oxide. Influence of the Power Output and the Nature of the Acylating Agent on the Selectivity." J. Org. Chem., Vol.61, No.16, (1996), p.5264-5270, Especially see p.5267, Scheme 6, Compound No.14 | 1-6,8,9 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 June, 2000 (23.06.00) | 04 July, 2000 (04.07.00) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No. PCT/JP00/02470

C (Continuation). DOCUMENTS CONSIDERED TO BE RELEVANT

| Category* | Citation of document, with indication, where appropriate, of the relevant passages | Relevant to claim No. |
|---|---|---|
| X | KLIEGEL Wolfgang, et al., "Ring-Chain isomerism of N-(2-hydroxyalkyl)nitrones. III. Nitorones of 2-furancarboxy-aldehydes." Chem. Ber., Vol.116, No.1, (1983), pp.27-45, Compounds No.14 and Database CA on STN, AMERICAN CHEMICAL SOCIETY (ACS),(Columbus, OH, USA), Compounds in AN.98:125787, (1983) | 1-4,8,9 |
| X | BERNABE M., et al., "Cycopropane derivatives. II. Spiro-oxazalones. Hydrolytic ring-opening reactions." An. Quim., Vol.68, No.7-8, (1972), p.1005-15, & Database CA on STN, AMERICAN CHEMICAL SOCIETY (ACS), (Columbus, OH,USA), AN.78:3760, (1973), Compounds in RN:39645-16-4 | 1,2,4-6,8,9 |
| X | KOZHIN S.A., et al., "Products of the aminolysis of limonene 1, 2-monoxide." Zh. Obshch. Khim., Vol.48, No.1, (1978), p.203-217, & Database CA on STN, AMERICAN CHEMICAL SOCIETY (ACS), (Columbus, OH, USA), AN.88:191084, (1978), Compounds in RN:66378-89-0 | 1-6,8,9 |
| X | WO, 98/54140, A1 (Dongwha Pharmaceutical Ind. Co., Ltd.), 03 December, 1998 (03.12.98), Abstract, Claims, Compound No.34, & US, 5922871, A & KR, 98080516, A & KR, 98086363 & KR, 99000415, A | 1-4,6,8,9 |
| A | GB, 1495286, A (Hoechst Aktiengesellschaft), 14 December, 1977 (14.02.77), Full text; especially see, Claim 10 (formula II), & JP, 50-89363, A & US, 3985889, A & US, 3962259, A & NL, 7415940, A & IL, 46202, A & DK, 7406450, A & CA, 1051886, A & AT, 7409883, A & CH, 620214, A & BE, 823279, A & FR, 2254332, A & Database CA on STN, AMERICAN CHEMICAL SOCIETY (ACS), (Columbus, OH, USA), AN.86:55302, (1977), Compounds in RN:56658-09-4, RN:54596-21-3, RN:56658-05-0, RN:56658-13-0 | 1-22 |

Form PCT/ISA/210 (continuation of second sheet) (July 1992)